# EUROPEAN PATENT APPLICATION

(11) **EP 1 159 937 A1**
(43) Date of publication of application: **05.12.2001**
(21) Application number: 00201893.5
(22) Date of filing: 29.05.2000
(51) Int. Cl.: A61F 2/36

(54) **A femoral prosthesis**

(71) Applicant: Spike Trading Lda, 9000 Funchal, Madeira (PT)
(72) Inventor: Travassos, Luis, 9000 Funchal Madeira (PT)
(74) Representative: Long, Giorgio

(57) **Abstract**

A femoral prosthesis (1) which can be implanted unusually easily and securely comprises a first, diaphysis prosthesis element (2) which can be housed in a medullary canal of a femur, and a second, metaphysis prosthesis element (16) which can be coupled releasably with the first prosthesis element and has an epiphysis portion (19) reproducing a neck (20) of a femur head which can be coupled releasably with a joint head (21). The first prosthesis element has a solid proximal end portion (3) provided externally with fixing means (8) and shaped coupling means (10) for coupling with the second prosthesis element in a plurality of predetermined angular positions, and the second prosthesis element has a seat (25) for housing the solid proximal portion of the first prosthesis element, the seat having complementary shaped coupling means (26) suitable for housing the shaped coupling means; complementary fixing means (34) can be coupled with the fixing means so as to fix the second prosthesis element firmly to the first prosthesis element.

## Description

The subject of the present invention is a femoral prosthesis, otherwise known as a prosthetic hip structure.

More particularly, the invention relates to a femoral prosthesis comprising a first, diaphysis prosthesis element which can be housed in a medullary canal of a femur and a second, metaphysis prosthesis element which can be coupled releasably with the first prosthesis element and has an epiphysis portion reproducing a neck of a femur head which can be coupled releasably with a joint head.

As is known, femoral prostheses of the type described above are adapted to various implantation conditions by virtue of the provision of separate prosthesis elements for coupling with the femur and for forming the hip joint. It is thus possible, for example, to implant a femoral prosthesis having a first, diaphysis prosthetic element of different proportions to a second, metaphysis prosthesis element in order to adapt the femoral prosthesis better to the patient's anatomy.

There is a great need to adapt the prosthesis to different operative morphology not only for first implantation operations but also for further operations on a limb already provided with a prosthesis (revision operations), as well as for operations on dysplasic hips.

However, it must be borne in mind that the provision of femoral prostheses of the type described above results in a need to form a secure connection between the prosthesis elements and, in particular, a connection which can withstand the stresses transmitted by the neck to the metaphysis prosthesis element and hence to the diaphysis prosthesis element connected to the femur, thus permitting normal use of the limb.

This need has been fulfilled in current femoral prostheses by virtue of connection means of considerable dimensions. However, this arrangement clearly has many disadvantages such as, for example, the need to provide prosthesis elements which can house the bulky connection means completely in order to avoid alterations to the natural morphology of the joint.

Moreover, the need to house connection means of considerable dimensions makes known prosthesis elements bulky and not suitable for use in all operative morphologies and, where they are usable, makes known prostheses particularly invasive.

The problem upon which the present invention is based is that of proposing a femoral prosthesis which has structural and functional characteristics such as to satisfy the above-mentioned needs and, at the same time, to overcome the disadvantages mentioned with reference to the prior art.

This problem is solved by means of a femoral prosthesis comprising a first, diaphysis prosthesis element which can be housed in a medullary canal of a femur and a second, metaphysis prosthesis element which can be coupled releasably with the first prosthesis element and which has an epiphysis portion reproducing a neck of a femur head which can be coupled removably with a joint head, characterized in that the first prosthesis element has a solid proximal end portion provided externally with fixing means and shaped coupling means for coupling with the second prosthesis element in a plurality of predetermined angular positions, in that the second prosthesis element has a seat for housing the solid proximal portion of the first prosthesis element, the seat having complementary shaped coupling means suitable for housing the shaped coupling means, and in that complementary fixing means can be coupled with the fixing means so as to fix the second prosthesis element firmly to the first prosthesis element.

Further characteristics and the advantages of the femoral prosthesis according to the invention will become clear from the following description of a preferred embodiment thereof, given by way of non-limiting example, with reference to the appended drawings, in which:
Figure 1 is a perspective view of a femoral prosthesis according to the present invention, with parts separated,
Figure 2 is a perspective view of the prosthesis of Figure 1, on an enlarged scale,
Figure 3 is a cross-section of the prosthesis of Figure 1, with parts separated,
Figure 4 is a cross-section of the prosthesis of Figure 1,
Figure 5 is a side view of a detail of a femoral prosthesis according to a second embodiment of the invention,
Figure 6 is a section of the detail of Figure 5, taken on the line VI-VI,
Figure 7 is a side view of a detail of a femoral prosthesis according to a third embodiment of the invention,
Figure 8 is a cross-section of a femoral prosthesis according to a further embodiment, with parts separated.

With reference to the drawings, a femoral prosthesis is generally indicated 1. The femoral prosthesis 1 comprises a first, diaphysis, shank, or root prosthesis element 2 having a proximal end portion, generally indicated 3, an intermediate proximal portion 4, and a distal portion 5. These portions extend along a axis X-X of the prosthesis so that they can be inserted, at least partially, in a medullary canal of a femur.

The proximal end portion 3 is advantageously solid. According to one embodiment of the invention, this portion comprises a pin 6 of limited transverse dimensions sufficient to withstand the stresses transmitted to the femoral prosthesis 1 during the use of a limb in which it has been implanted. The pin 6 has an end surface 7 which is free of recesses, for example, a slightly inclined or sloping surface. The solid, proximal end portion 3 has external fixing means 8. According to one embodiment, the fixing means comprise a thread 9 formed externally on the pin 6. The external thread 9 is provided on a proximal end section of the pin 6. A second, distal section of the pin 6 has shaped coupling means 10. According to one embodiment, the distal section of the pin comprises a plurality of longitudinal grooves 11 formed in the surface of a portion of the distal section which, for example, is cylindrical in shape and/or is defined at its proximal end by a reference or locating surface 12. These longitudinal grooves are, for example, distributed uniformly around the pin and/or are spaced apart uniformly, defining predetermined angular positions relative to the axis X-X of the prosthesis.

The distal end of the pin 6 adjoins a first section 13 of the intermediate proximal portion 4. This first section 13 advantageously has a frustoconical surface. The frustoconical section is connected to a second, straight proximal section 14, for example, having a cylindrical surface.

According to one embodiment of the invention, the distal portion of the first, diaphysis prosthesis element is straight, for example, with a tapered shape extending from the intermediate proximal portion 4 to the distal end 15.

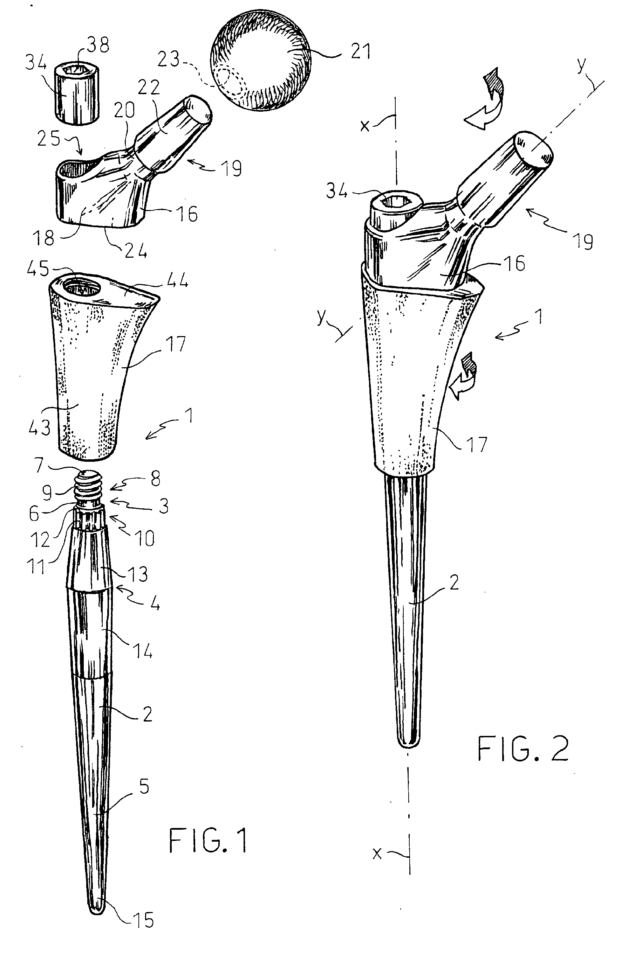

The femoral prosthesis 1 also comprises a second, metaphysis prosthesis element 16 or turret. The second, metaphysis prosthesis element 16 has a body 18 from which an epiphysis portion 19 reproducing a neck 20 of a femur head extends along an axis Y-Y. The neck 20 can be coupled releasably with a joint head 21 by means of a proximal portion or pin 22 (Figure 1). This proximal portion has a shape matching that of a seat 23 provided in the joint head. For example, the pin 22 and the seat 23 are frustoconical. The body 18 is of a limited size or, in other words, it has dimensions such that it can support the epiphysis portion firmly and can transmit the stresses coming from the hip to the first, diaphysis prosthesis element 2 and, at the same time, such that it is extremely compact. The distal portion of the body 18 is defined by a reference or locating surface 24 which faces the first, diaphysis prosthesis element 2. In the body, there is a seat 25 for housing the solid proximal end portion of the diaphysis prosthesis element. The seat 25 has complementary shaped coupling means 26 which can be coupled with the shaped coupling means 10 provided on the solid proximal end 3 of the diaphysis element 2. According to one embodiment, the seat 25 comprises a through-hole 27 having a first, distal section 28 with transverse dimensions smaller than those of a second, proximal section 29. A plurality of longitudinal projections 30 of a shape matching that of the plurality of longitudinal grooves 11 provided on the pin 6 of the first, diaphysis prosthesis element 2 extends from at least a portion of the surface of the first distal section 28. The plurality of projections 30 can be coupled with the plurality of grooves 11 in a manner such that the second, metaphysis prosthesis element 16 can be arranged in a predetermined angular position relative to the axis X-X of the first, diaphysis prosthesis element 2. Advantageously, the plurality of projections 30 is provided in the distal end portion of the through-hole 27 and is defined at its proximal end by an annular rim or projection 31 forming an abutment surface 32 for the locating surface 12 provided on the pin 6. The wider, second proximal section 29 of the through-hole 27 has, for example, a circular cross-section and constitutes a seat 33 for complementary fixing means 34 which can be coupled with the fixing means 8 provided in the pin 6 so as to fix the second, metaphysis prosthesis element 16 firmly to the first prosthesis element 2. According to one embodiment, the complementary fixing means 34 comprise a nut 35 having a threaded hole 36. In particular, the nut has a threaded hole 36 and, at the opposite end 37, a seat 38 for housing operating means usable for tightening the nut when it is engaged with the pin 6. For example, the seat is shaped for housing a hexagonal wrench (an Allen key). The hole 36 is preferably a through-hole or, in other words, the hexagonal seat is in communication with the threaded hole through an abutment rim, thus avoiding accumulation of material in the nut before or during implantation. According to one embodiment, the nut 35 can be inserted almost completely in the seat 33. The base of the seat has an annular abutment surface 39 for the nut 34.

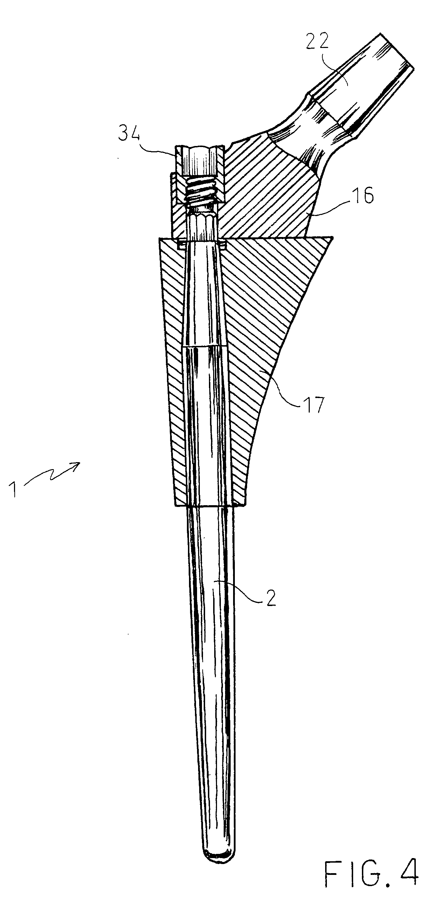

In addition to the prosthesis elements described above, according to an advantageous embodiment of the invention, the femoral prosthesis 1 comprises a further prosthesis element. The further element is an intermediate metaphysis prosthesis element 17. This intermediate element 17 is formed in a manner such that it can be fitted on the intermediate proximal portion 4 of the first, diaphysis prosthesis element 2 (Figure 4). The intermediate element 17 has a tapered body 40 extending from a proximal end 41 of the element to a distal end 42 thereof. In particular, the intermediate element 17 is shaped in a manner such as to fill the space formed in the metaphysis portion of the femur in order to house the prosthesis, as well as distributing better the stresses transmitted by the hip through the prosthesis, to the femur. The body advantageously has outer surfaces 43 with a coarse roughness or other treatment which promotes osteointegration. The proximal end 41 of the body is defined by an abutment surface 44 for receiving the locating surface of the second, metaphysis prosthesis element 16. In the body of the intermediate element 17, there is a seat shaped so as to enable the intermediate element 17 to be coupled with the intermediate proximal portion 4 of the first, diaphysis prosthesis element 2. According to one embodiment, a through-hole 45 in the body 40 of the intermediate element 17 is formed by a proximal portion 46 with a frustoconical surface and a straight portion 47. The portion 46 with the frustoconical surface can be coupled with the frustoconical portion 13 of the first element 2 which constitutes an abutment and defines a predetermined longitudinal position of the intermediate element 17 relative to the first element 2. The above-described coupling between the intermediate element 17 and the first element 2 also enables the intermediate element 17 to be positioned freely angularly relative to the first prosthesis element 2. In particular, the portion 46 of the through hole having the frustoconical surface is arranged in the body 40 in a manner such as to house the first element 2, causing the solid proximal end portion 3 of the first element 2 to project at least partially from the proximal end 41 of the body 40. The at least partial projection of the solid portion 3 should be such as to allow the solid portion 3 to be housed by the complementary angular positioning means 26 and to engage the complementary fixing means 34 of the second element 16. According to one embodiment, the through-hole 45 has, at its proximal end, an enlarged portion 48 which, when the first prosthesis element 2 is housed in the through-hole in the working position, is disposed adjacent a portion of the shaped coupling means 10 which is not engaged by the complementary shaped coupling means 26 provided in the second element 17.

Some steps of an operation to implant the femoral prosthesis of the invention are described below.

Once the femur or the portion thereof which is to receive the femoral prosthesis has been prepared, the first diaphysis prosthesis element is inserted in the medullary canal so as to be fixed firmly to the bone and to form a support structure. During this stage of the operation it will not be necessary to pay attention to its angular arrangement relative to the acetabulum since the metaphysis prosthesis elements and, in particular, the second metaphysis prosthesis element having the neck, can be connected to the first prosthesis element in a manner such that their angular arrangement is adjustable. It is therefore necessary to pay attention solely to the correct depth of positioning of the first diaphysis prosthesis element in the medullary canal, greatly simplifying this first step of the operation which is particularly laborious *per se.*

The intermediate metaphysis element is then fitted until it is brought into abutment with the frustoconical section of the first, diaphysis prosthesis element. During the coupling of this intermediate element, it is possible to adjust the angular position of the intermediate metaphysis element precisely so as to cause a large part of its body to fit closely against the walls of the seat formed in the femur and to ensure that a large surface of the prosthesis bears on the femur, ensuring adequate distribution of the stresses transmitted to the femur by the prosthesis.

The above-described steps of the operation result in the formation of residues of organic and non-organic material which are deposited on the coupling surfaces of the prosthesis elements. By virtue of the provision of the solid proximal end portion of the first diaphysis prosthesis element, the above-mentioned residues are easily removed so as to permit subsequent coupling with the seat of the second, metaphysis prosthesis element. Only by rapid and complete removal of the residues is a perfect coupling of the shaped coupling means and the complementary shaped coupling means ensured.

The second, metaphysis prosthesis element is at least partially outside the femur bone and is positioned angularly relative to the first, diaphysis prosthesis element (of axis X-X) in a manner such that its epiphysis portion (of axis Y-Y), and hence the joint head, is arranged perfectly in alignment with the acetabulum of the hip. This step is also facilitated by the limited dimensions or compactness of the second, metaphysis prosthesis element.

The prosthesis elements are thus clamped together by connecting to the threaded pin of the first prosthesis element the complementary fixing means which clamp the second and intermediate metaphysis prosthesis elements against the first element as an assembly.

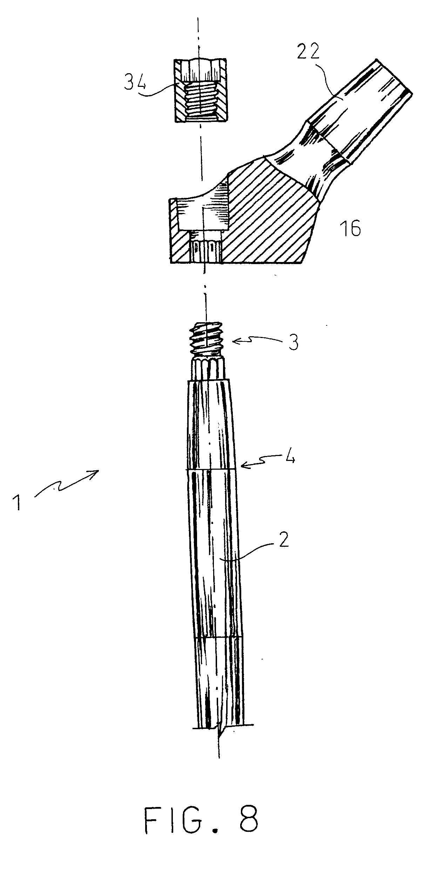

As an alternative to the method described above, for example, for a first implant in which a lesion of the femur is not very extensive, the second, metaphysis prosthesis element is connected directly to the first, diaphysis prosthesis element, without the interposition of the intermediate metaphysis prosthesis element (Figure 8).

As can be appreciated from the foregoing description, the femoral prosthesis according to the present invention satisfies the above-mentioned need to connect the diaphysis prosthesis element firmly to the metaphysis prosthesis element without any need to provide connection means of appreciable size or, all the less, to provide bulky and invasive prosthesis elements. In fact, by virtue of the solid proximal end portion, the proposed first, diaphysis prosthesis element has small dimensions and can thus easily be housed in the seat of the second, metaphysis prosthesis element which, as a result, is also of a compact size.

By virtue of the small size of the prosthesis elements, as well as obtaining a femoral prosthesis which is strong and at the same time not very invasive, it is possible to adapt the femoral prosthesis of the invention morphologically to the most varied pathological situations.

It is in fact an advantage that, by virtue of the shaped coupling means and the complementary shaped coupling means, the femoral prosthesis of the invention permits a large degree of adjustment of the angular position of the neck relative to the first, diaphysis prosthesis element, at the same time preventing relative rotation thereof.

The various functions required of the hip joint are advantageously performed in the femoral prosthesis of the invention by distinct prosthesis elements. In particular, in the proposed femoral prosthesis, the load-bearing structural function is performed by the first, diaphysis prosthesis element, whilst the function of connection to the hip and support of the joint head is performed by the second, metaphysis prosthesis element.

The provision of distinct prosthesis elements for performing distinct functions, together with connection means which permit a precise adjustment of the relative arrangement of these elements, enables a very considerable and varied range of pathological conditions to be resolved by a limited number of prosthesis elements having different shapes or sizes. In other words, by virtue of the provision of distinct prosthesis elements, a high degree of modularity or a high degree of adaptability to the morphology of the patient's joint is advantageously achieved. For example, it is possible to construct a femoral prosthesis by alternatively connecting metaphysis prosthesis elements having different shapes to a predetermined first, diaphysis prosthesis element selected so as to be perfectly adapted to the shape of a medullary canal, until the functionality of the joint is correctly re-established.

The provision of a solid proximal end portion of the first, diaphysis prosthesis element achieves a further advantage. By virtue of this portion, the operations to connect the prosthesis elements are easy, quick and precise. In fact, the solid end portion constitutes a guide element which facilitates the correct positioning and connection of the second and intermediate metaphysis elements during implantation. Moreover, the provision of a solid pin prevents accumulation of biological material on the proximal end of the diaphysis prosthesis element during the insertion of this element in the medullary canal, which would render the operations to fit the metaphysis elements laborious and the connection between these elements and the diaphysis element insecure. The security of the connection between the prosthesis elements also renders the implantation achieved by the proposed prosthesis durable over time.

In particular, the proposed prosthesis can be fitted extremely quickly, by virtue of the provision of complementary fixing means which can be engaged with the external fixing means provided on the solid end portion to fix the metaphysis elements. Moreover, the external fixing means provided on the solid proximal end portion can easily be cleaned before they are connected to the complementary fixing means, simplifying the process of connecting the elements and, above all, preventing the occurrence of infections caused by accumulation of material between the prosthesis elements.

A further advantage of the femoral prosthesis according to the invention lies in its unusual structural simplicity which enables it to be produced at a low cost.

Clearly, variants and/or additions may be provided for the embodiments described and illustrated.

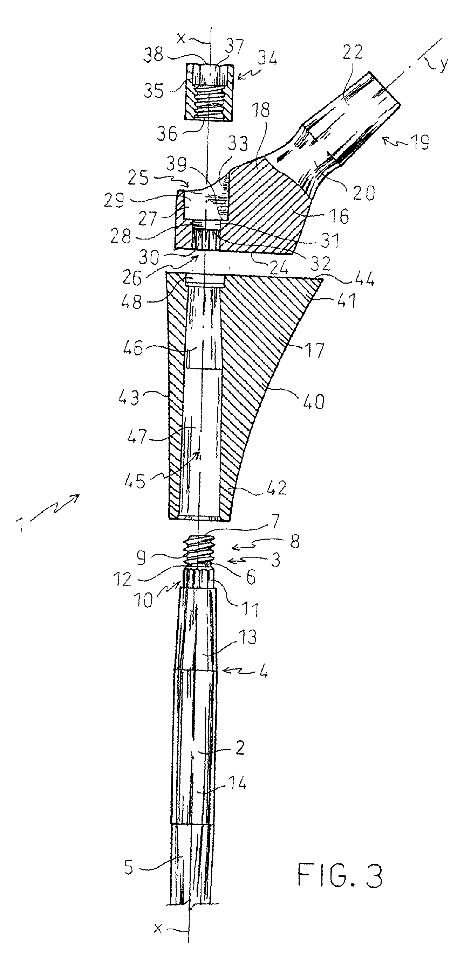

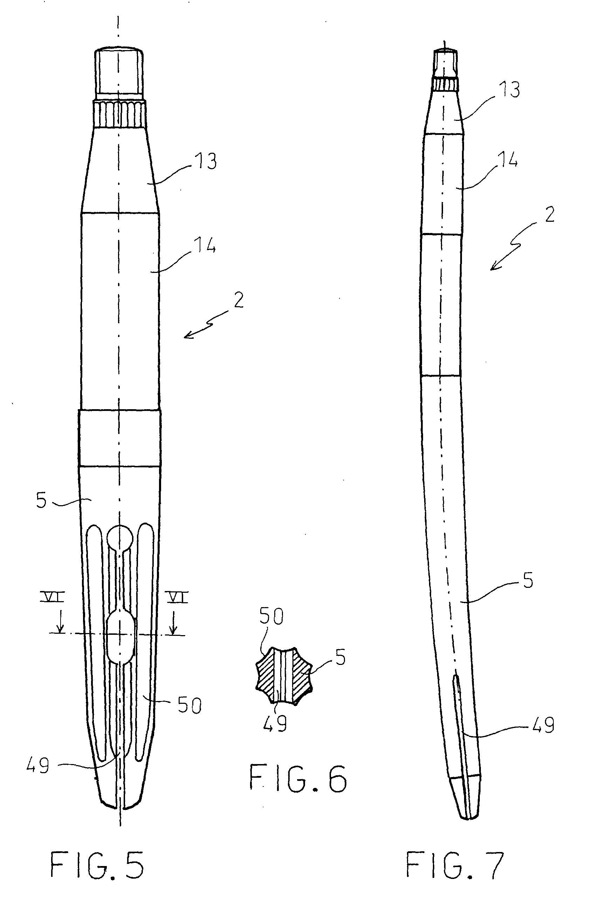

As an alternative to the embodiment shown in Figures 1 to 4, the first, diaphysis prosthesis element 2 may have its distal portion 5 slightly curved (Figure 7).

Moreover, at least one longitudinal slot 49 may be provided in the distal end 15 of the first element (Figures 5 and 7).

According to yet a further alternative, the first prosthesis element 2 has a distal portion having at least one groove 50 which permits better coupling between the element 2 and the walls of the medullary canal (Figures 5 and 6).

As an alternative to the embodiment shown in Figures 1, 3 and 4, the pin 6 of the first element 2 has projections for shaped coupling with grooves provided in the seat 25 of the second element 16.

In order to satisfy contingent and specific requirements, an expert in the art may apply to the above-described preferred embodiment of the femoral prosthesis many modifications, adaptations and replacements of elements with other functional equivalent elements, without however departing from the scope of the following claims.

## Claims

1. A femoral prosthesis (1) comprising a first, diaphysis prosthesis element (2) which can be housed in a medullary canal of a femur, and a second, metaphysis prosthesis element (16) which can be coupled releasably with the first prosthesis element and has an epiphysis portion (19) reproducing a neck (20) of a femur head which can be coupled releasably with a joint head (21), **characterized in that** the first prosthesis element has a solid proximal end portion (3) provided externally with fixing means (8) and shaped coupling means (10) for coupling with the second prosthesis element in a plurality of predetermined angular positions, **in that** the second prosthesis element has a seat (25) for housing the solid proximal portion of the first prosthesis element, the seat having complementary shaped coupling means (26) suitable for housing the shaped coupling means, and **in that** complementary fixing means (34) can be coupled with the fixing means so as to fix the second prosthesis element firmly to the first prosthesis element.

2. A prosthesis according to Claim 1, in which the solid proximal end portion comprises a pin (6).

3. A prosthesis according to Claim 2, in which the fixing means comprise a thread (9) formed externally on the pin.

4. A prosthesis according to Claim 3, in which the complementary fixing means comprise a nut (35) having a threaded hole (36).

5. A prosthesis according to Claim 4, in which the nut comprises a seat (38) for housing operating means usable for tightening the nut.

6. A prosthesis according to any one of the preceding claims, in which the second prosthesis element comprises a seat (33) for housing the complementary fixing means.

7. A prosthesis according to Claim 6, in which the seat comprises a surface (39) for abutment by the complementary fixing means in the tightened position.

8. A prosthesis according to any one of the preceding claims, in which the complementary shaped coupling means comprise at least a longitudinal projection (30).

9. A prosthesis according to Claim 8, in which the shaped coupling means comprise a plurality of longitudinal grooves (11).

10. A prosthesis according to Claim 8 or Claim 9, in which the plurality of longitudinal grooves is provided on a section of the solid portion which is defined at the proximal end by a locating surface (12).

11. A prosthesis according to Claim 9 or Claim 10, in which the complementary shaped coupling means have an abutment surface (32) for the locating surface.

12. A prosthesis according to any one of the preceding claims, in which an intermediate metaphysis prosthesis element (18) is provided and can be coupled releasably with an intermediate proximal portion (4) of the first prosthesis element.

13. A prosthesis according to Claim 12, in which the intermediate prosthesis element has a through-hole (45) having a portion with a frustoconical surface (46) which can be coupled with a frustoconical portion (13) of the intermediate proximal portion of the first prosthesis element for precise longitudinal positioning and free angular positioning of the intermediate element relative to the first prosthesis element.

14. A prosthesis according to Claim 13, in which the through-hole houses the first prosthesis element in a manner such that the solid proximal end portion projects partially from the intermediate prosthesis element in order to be housed in the angular positioning and fixing seat of the second prosthesis element.

15. A prosthesis according to Claim 13 or Claim 14, in which the through-hole has an enlarged portion (48) which is disposed adjacent the shaped coupling means when the first prothesis element is housed in the through-hole, in the working position.

16. A prosthesis according to any one of Claims 12 to 15, in which the intermediate prosthesis element has an abutment surface (44) for the second prosthesis element.

17. A prosthesis according to any one of the preceding claims, in which at least one of the first, second and intermediate prosthesis elements has an outer surface (43) with coarse roughness to promote osteointegration.

18. A prosthesis according to any one of the preceding claims, in which the first prosthesis element has a straight proximal portion (13, 14) and a slightly curved distal portion (5).

19. A prosthesis according to any one of the preceding claims, in which the first prosthesis element has a distal section (5) having at least one groove (50).

20. A prosthesis according to any one of the preceding claims, in which the first prosthesis element has a distal end (5) having at least one longitudinal slot
